# EUROPEAN PATENT SPECIFICATION

(11) **EP 3 561 079 B1**
(45) Date of publication and mention of the grant of the patent: **21.08.2024**
(21) Application number: 19170389.1
(22) Date of filing: 19.04.2019
(51) Int. Cl.: C12Q 1/6895, A01H 6/54

(54) **SOYBEAN ANTI-POD-SHATTERING MAJOR QTLQPD08-1, AND MAPPING METHOD AND APPLICATION THEREOF**
MAJOR QTLQPD08-1 ZUM SCHUTZ VOR DEM HÜLSENPLATZEN BEI SOJABOHNEN SOWIE MAPPING-VERFAHREN UND ANWENDUNG DAVON
QTLQPD08-1 MAJEUR ANTI-ÉGRENAGE DE GOUSSES DE GRAINES DE SOJA ET PROCÉDÉ DE MISE EN CORRESPONDANCE ET SON APPLICATION

(30) Priority: 24.04.2018 CN 201810374345
(43) Date of publication of application: 30.10.2019
(73) Proprietor: Institute of Crop Sciences, Chinese Academy of Agricultural Sciences, Beijing 100081 (CN)
(72) Inventor: QIU, Lijuan, Beijing (CN); HAN, Dezhi, Beijing (CN); HAN, Jianan, Beijing (CN); YAN, Hongrui, Beijing (CN); SU, Bohong, Beijing (CN)
(74) Representative: Iannone, Carlo Luigi

(56) References cited:
- HIDEYUKI FUNATSUKI ET AL: "Mapping and use of QTLs controlling pod dehiscence in soybean", BREEDING SCIENCE, vol. 61, no. 5, 1 January 2012 (2012-01-01), pages 554 - 558, XP055216552, ISSN: 1344-7610, DOI: 10.1270/jsbbs.61.554
- XIAOWEN SUN ET AL: "SLAF-seq: An Efficient Method of Large-Scale De Novo SNP Discovery and Genotyping Using High-Throughput Sequencing", PLOS ONE, vol. 8, no. 3, 19 March 2013 (2013-03-19), pages e58700, XP055622605, DOI: 10.1371/journal.pone.0058700
- BIN LI ET AL: "Construction of a high-density genetic map based on large-scale markers developed by specific length amplified fragment sequencing (SLAF-seq) and its application to QTL analysis for isoflavone content in Glycine max", BMC GENOMICS, BIOMED CENTRAL, vol. 15, no. 1, 10 December 2014 (2014-12-10), pages 1086, XP021208975, ISSN: 1471-2164, DOI: 10.1186/1471-2164-15-1086
- CAO YONGCE ET AL: "Mapping QTLs for plant height and flowering time in a Chinese summer planting soybean RIL population", EUPHYTICA, KLUWER ACADEMIC PUBLISHERS, NL, vol. 213, no. 2, 9 January 2017 (2017-01-09), pages 1 - 13, XP036154010, ISSN: 0014-2336, [retrieved on 20170109], DOI: 10.1007/S10681-016-1834-8

### Sequence Listing

<110> Institute of Crop Sciences, Chinese Academy of Agricultural Sciences
<120> SOYBEAN ANTI-POD-SHATTERING MAJOR QTLqPD08-1, AND MAPPING METHOD AND APPLICATION THEREOF, AND SOYBEAN ANTI-POD-SHATTERING GENE AND APPLICATION THEREOF
<160> 54
<170> SIPOSequenceListing 1.0
<210> 1
<211> 24
<212> DNA
<213> Artificial Sequence
<400> 1
   tctcatgacc acaaacgagt ccct 24
<210> 2
<211> 25
<212> DNA
<213> Artificial Sequence
<400> 2
   ggtcttggtg accttgacca tatgg 25
<210> 3
<211> 229
<212> DNA
<213> Artificial Sequence
<400> 3
   catttttcag aaccccgatc tgatgaacaa ggtgcgtgat ttgttcaact tcaacaacaa 60
   caacaacaac aacaaccctg aaacgtgttc ctgggcgttg aattgcgttg ccaccaccga 120
   tcaaggcgag aacgaccctt cctcgctctg gctcaaccct gaaatcaaag actcctccac 180
   cgtctcgccg ccgaattcca cggttaataa gacgatgcat ttcgaaacc 229
<210> 4
<211> 93
<212> DNA
<213> Artificial Sequence
<400> 4
   gcagttgttg caattggaag atgttcagaa aatgaaatac tcttggaatg ttgcatctga 60
   agtcatgagg ctatccctac ctgtgagtgg tgc 93
<210> 5
<211> 22
<212> DNA
<213> Artificial Sequence
<400> 5
   catttttcag aaccccgatc tg 22
<210> 6
<211> 22
<212> DNA
<213> Artificial Sequence
<400> 6
   ggtttcgaaa tgcatcgtct ta 22
<210> 7
<211> 25
<212> DNA
<213> Artificial Sequence
<400> 7
   gcagttgttg caattggaag atgtt 25
<210> 8
<211> 23
<212> DNA
<213> Artificial Sequence
<400> 8
   gcaccactca caggtaggga tag 23
<210> 9
<211> 22
<212> DNA
<213> Artificial Sequence
<400> 9
   ttctccaacc tctcagtttc tc 22
<210> 34
<211> 22
<212> DNA
<213> Artificial Sequence
<400> 10
   aacagtggtc gataacaaga ct 22
<210> 11
<211> 23
<212> DNA
<213> Artificial Sequence
<400> 11
   tatcagactc aatgggaaaa cgg 23
<210> 12
<211> 23
<212> DNA
<213> Artificial Sequence
<400> 12
   ttgcagataa aaccgcgcca aaa 23
<210> 13
<211> 22
<212> DNA
<213> Artificial Sequence
<400> 13
   gtatcagcag catacccttt tg 22
<210> 14
<211> 22
<212> DNA
<213> Artificial Sequence
<400> 14
   ctgtctttct cctcaacttt gc 22
<210> 15
<211> 22
<212> DNA
<213> Artificial Sequence
<400> 15
   gaatccgcat tcaacatgaa ga 22
<210> 16
<211> 22
<212> DNA
<213> Artificial Sequence
<400> 16
   ggattgaatg gagttgacat cg 22
<210> 17
<211> 22
<212> DNA
<213> Artificial Sequence
<400> 17
   aaaggttttt ggccctgtat tc 22
<210> 18
<211> 22
<212> DNA
<213> Artificial Sequence
<400> 18
   taggaaaatc gtaaggccaa ct 22
<210> 19
<211> 23
<212> DNA
<213> Artificial Sequence
<400> 19
   taggagacaa ggtgggtgtg gga 23
<210> 20
<211> 25
<212> DNA
<213> Artificial Sequence
<400> 20
   gctatacact gtgatcccag cacag 25
<210> 21
<211> 23
<212> DNA
<213> Artificial Sequence
<400> 21
   tcgtgatgtg gttatttgaa caa 23
<210> 22
<211> 23
<212> DNA
<213> Artificial Sequence
<400> 22
   aaggcgaaga gatggaagag agt 23
<210> 23
<211> 22
<212> DNA
<213> Artificial Sequence
<400> 23
   tgatgttata aatcccgcaa cg 22
<210> 24
<211> 22
<212> DNA
<213> Artificial Sequence
<400> 24
   gcactaactg cagctttaaa ct 22
<210> 25
<211> 22
<212> DNA
<213> Artificial Sequence
<400> 25
   gtgctttaag ccaagtcttt ca 22
<210> 26
<211> 22
<212> DNA
<213> Artificial Sequence
<400> 26
   gttatcggct tcggtttttc tt 22
<210> 27
<211> 22
<212> DNA
<213> Artificial Sequence
<400> 27
   aatagtgctc tctctcacac ac 22
<210> 28
<211> 22
<212> DNA
<213> Artificial Sequence
<400> 28
   gtgctcaaac ttgcagttgt aa 22
<210> 29
<211> 20
<212> DNA
<213> Artificial Sequence
<400> 29
   ggtggttcta tcttggcatc 20
<210> 30
<211> 20
<212> DNA
<213> Artificial Sequence
<400> 30
   ctttcgcttc aataacccta 20

## Description

### TECHNICAL FIELD

The present invention belongs to the field of QTL mapping, and particularly relates to a soybean anti-pod-shattering gene obtained by screening based on the soybean anti-pod-shattering major *QTLqPD08-1*, and primers for amplifying the molecule marker of soybean anti-pod-shattering major *QTLqPD08-1.*

### BACKGROUND

Pod shattering is essential for the seed dispersal of a wild plant having pods. For a wild plant, seed dispersal is a necessary process for the survival and reproduction of the wild plant, which provides the future generations with sufficient growth space and the chance to survive under different environmental conditions (Fuller 2007). However, in cultivated crops, pod shattering results in severe yield reduction, since seeds cannot be harvested due to pod shattering. During planting and harvesting by farmers, it is desirable to retain pods prior to harvesting, and anti-pod shattering is a trait of unconscious selection associated with crop yield (Hancock and Hancock 2003; Harlan *et al.* 1973). Therefore, anti-pod shattering is an important domestication trait during crop domestication (Hideyuki *et al.* 2012). Although we strictly screened during crop domestication to avoid pod shattering, pod shattering before harvesting is still a problem that needs to be addressed urgently in breeding (Christiansen *et al.* ). Although crop anti-pod-shattering have made great progress in genetic breeding, we know little about the gene for crop anti-pod-shattering (Funatsuki *et al.* 2014).

Over the past 20 years, a series of QTL loci associated with soybean pod shattering have been identified. So far, a large number of studies have used RFLP (Bailey *et al.* 1997), SSR markers (Funatsuki *et al.* 2006) and the like methods to map QTLs associated with soybean pod shattering by means of recombinant inbred lines, indicating that soybean pod shattering is controlled by the major QTL and some minor QTLs. Since different genes control pod shattering due to the different genetic backgrounds of a population, the anti-pod-shattering characteristic is embodied as controlled by a single recessive major gene or multiple genes (Kang *et al*.2005). Bailey *et al.* first discovered a major QTL existed on chromosome 16 using recombinant inbred lines (RILS) and found some minor QTLs respectively on chromosomes 2, 15, and 19 (Bailey *et al.*1997), and after fine mapping, the candidate gene Glyma16g25580 controls the pod shattering and is named as Pdh1 (Pod dehiscence 1) (Funatsuki *et al.* 2014). At the same time, the study found that a major NAC gene in soybean, i.e., Glyma.16g019400, named as SHAT1-5, also regulates soybean pod shattering (Dong *et al.* 2014). Through two sets of RILs, Kang *et al.* remapped three new minor QTLs on chromosomes 5, 10 and 14 using an SSR marker (Kang *et al.* 2009).

Hideyuki Funatsuki *et al.* (Hideyuki Funatsuki et al.: "Mapping and use of QTLs controlling pod dehiscence in soybean" Breeding Science, vol 61, no 5, 1 January 2012 (2012-01-01), pages 554-558, XP055216552, SSN: 1344-7610, DOL: 10.1270/sbbs. 61. 554) disclose SNP molecular marker used in kit for high-throughput for controlling pod dehiscence in soybean. Xiaowen Sun *et al.* (Xiaowen Sun et al.: "SLAF-seq: An Efficient Method of Large-scale De Novo SNP Discovery and Genotyping Using High-throughput Sequencing", PLoS ONE, vol 8, no. 3, 19 March 2013 (2013-03-19), page e58700, XP055622605, Dol: 10. 1371/journal pone. 0058700) disclose the advantages of SLAF-seq technology for large scale genotyping. DBIN LI *et al.* (DBIN LI et al: "Construction of a high-density genetic map based on large-scale markers developed by specific length amplified fragment sequencing (SLAF-seq) and its application to QTL analysis for isoflavone content in Glycine max", BMC GENOMICS, BIOMED CENTRAL, vol. 15, no 1, 10 December 2014 (2014-12-10), page 1086, XP021208975, ISSN: 1471-2164, DOI: 10.1186/1471-2164-15-1086) disclose the use of SLAF-seq technology for the construction of a high-density genetic map and its application to QTL analysis for isoflavone content in soybean.

### SUMMARY

In view of this, an objective of the present invention is to provide a soybean anti-pod-shattering gene obtained by screening based on the soybean anti-pod-shattering major *QTLqPD08-1*, which may avoid pod shattering.

To achieve the above objective, the present invention provides the following technical solutions.

Primers for amplifying a molecule marker of soybean anti-pod-shattering major *QTLqPD08-1* are provided.

The present invention provides a soybean anti-pod-shattering major QTL*qPD08*-*1*, where the *qPD08-1* is mapped on the chromosome 8 of soybean at a physical position between 35560353-38482164; and
the genetic distance of 35560353-38482164 is 65.316-65.971 cM.

The present invention provides a method for mapping the soybean anti-pod-shattering major QTL*qPD08*-*1*, including the following steps:
(1) constructing recombinant inbred lines of the fifth and sixth generations by using pod-shattering soybean and anti-pod-shattering soybean as parents, simultaneously sowing the pod-shattering soybean, the anti-pod-shattering soybean and the recombinant inbred line of the fifth-generation, and simultaneously sowing the pod-shattering soybean, the anti-pod-shattering soybean and the recombinant inbred line of the sixth-generation, to obtain samples of the pod-shattering soybean, the anti-pod-shattering soybean and the soybean recombinant inbred lines in two years;
(2) respectively extracting DNAs from samples of the pod-shattering soybean, the anti-pod-shattering soybean and the soybean recombinant inbred lines in the two years, to obtain genomic DNAs of the pod-shattering soybean, the anti-pod-shattering soybean and the soybean recombinant inbred lines;
(3) respectively constructing SLAF libraries by utilizing the genomic DNAs of the pod-shattering soybean, the anti-pod-shattering soybean and the soybean recombinant inbred lines, and separately sequencing the constructed SLAF libraries to obtain original sequencing data;
(4) filtering the original sequencing data to select 4-103 bp in the middle of each reads as the analysis data;
(5) aligning the analysis data to a reference genome, where each reads that has paired ends aligned to a reads at the same position is of the same SLAF tag, and then conducting polymorphic analysis of the SLAF tags according to the differences in the number of alleles and the gene sequences to obtain polymorphic SLAF tags;
(6) filtering the polymorphic SLAF tags to obtain screened polymorphic SLAF tags;
   where the filtering is to delete the polymorphic SLAF tags having the following conditions:
   a. a polymorphic SLAF tag having a parent sequencing depth below 10X;
   b. a polymorphic SLAF tag containing more than 5 SNP loci; and
   c. a polymorphic SLAF tag that is insufficient to cover 70% of genotype individuals in all progenies;
(7) aligning the screened polymorphic SLAF tags with a reference genome for mapping, where the screened polymorphic SLAF tags are mapped to 20 chromosomes, and using each chromosome as a linkage group to calculate a genetic distance between adjacent polymorphic SLAF tags on each linkage group, so as to obtain a high-density genetic map; and
(8) conducting QTL mapping of the pod-shattering phenotypes and pod-shattering traits of soybeans in the two years by employing an inclusive composite interval mapping method according to the high-density genetic map, so as to obtain the interval *qPD08-1* of the soybean anti-pod-shattering major QTLs.

Preferably, in step (2), the number of the samples of soybean recombinant inbred lines in each of the two years independently meets the requirement of 200-300 samples.

Preferably, in step (1) the pod-shattering soybean is used as the male parent; and the anti-pod-shattering soybean is used as the female parent.

Preferably, the variety of the pod-shattering soybean is Heihe 18; and the variety of the anti-pod-shattering soybean is Heihe 43.

Preferably, in step (3), during the separate sequencing the sequencing depth of the SLAF library of the pod-shattering soybean is 39.74 X; the sequencing depth of the SLAF library of the anti-pod-shattering soybean is 34.87 X; and the sequencing depth of the SLAF library of the soybean recombinant inbred line is 12.35X.

Preferably, in step (4), the filtration criteria are as follows: filtering out a reads containing a linker sequence; and filtering out a reads having a N content exceeding 10% of the length of the reads.

Preferably, in step (7), before the calculation of the genetic distance, the method further includes performing second filtering on the screened polymorphic SLAF tags; where the criterion for the second filtering is to calculate an MLOD value between pairwise polymorphic SLAF tags, and then filter out polymorphic SLAF tags each with a MLOD value below 5.

The present invention provides an application of the above soybean anti-pod-shattering major QTL*qPD08-1* or a soybean anti-pod-shattering major QTL*qPD08*-*1* obtained by the above mapping method in studying the genetic mechanism of the pod-shattering trait or the screening for molecular markers related to soybean yield. The molecule marker is an Indel molecule marker; the primers for amplifying the Indel molecule marker include a forward primer and a reverse primer; the forward primer has a nucleotide sequence as shown in SEQ ID NO. 1 of the Sequence Listing; and
the reverse primer has a nucleotide sequence as shown in SEQ ID NO. 2 of the Sequence Listing.

The present invention provides the use of the primers in map-based cloning.

Use of the primers in the genetic mechanism of the pod-shattering trait or the screening for molecular markers related to soybean yield is provided;
wherein the major QTL is mapped on the chromosome 8 of soybean at a physical position between 35560353-38482164; and the genetic distance of 35560353-38482164 is 65.316-65.971 cM; selecting the soybean Williams 82 v2.1 genome as a reference genome.

The present invention provides a soybean anti-pod-shattering gene obtained by screening based on the soybean anti-pod-shattering major QTL*qPD08*-*1*, wherein the soybean anti-pod-shattering gene is a MYC2 transcription factor coding sequence or a saponin synthetase coding sequence;
where a nucleotide sequence of the MYC2 transcription factor is as shown in SEQ ID No. 3 of Sequence Listing; and
a nucleotide sequence of the saponin synthetase coding sequence is as shown in SEQ ID No. 4 of Sequence Listing.

The present invention provides the application of the soybean anti-pod-shattering gene or its modified version in selection of anti-pod-shattering varieties of soybean.

The present invention provides a soybean anti-pod-shattering major QTL*qPD08*-*1*, the *qPD08-1* is mapped on the chromosome 8 of soybean at a physical position between 35560353-38482164; and the genetic distance of 35560353-38482164 is 65.316-65.971 cM. Since the yield of soybean is positively correlated with the anti-pod shattering rate, it is possible to significantly improve the rate of anti-pod-shattering by detecting the soybean anti-pod-shattering major QTL and thus screening the varieties having the anti-pod-shattering major QTL. Therefore, mapping the major QTL*qPD08*-*1* is a breeding process for further detecting the anti-pod-shattering trait of soybean and thus improving the soybean yield and improving good soybean varieties. Meanwhile, the major QTL*qPD08*-*1* provided by the present invention is detected in planting of 2015 and 2016, indicating that the QTL has relatively better stability.

The present invention provides a method for mapping the soybean anti-pod-shattering major QTL*qPD08-1.* In the present invention a SLAF marker is screened at the whole genome level of the soybean by utilizing a SLAF-seq sequencing technology, so as to explore the QTLs related to pod shattering from this population. By using a material of a RIL7 population which has pod-shattering soybean and anti-pod-shattering soybean as the parents, a high-density genetic linkage map covering the whole genome of soybean is constructed, and QTL mapping of the anti-pod-shattering trait is carried out on this population to obtain QTLs related to anti-pod-shattering. In addition to this, the construction of the high-density genetic linkage map and the identification of the novel QTLs related to anti-pod-shattering specific to this population provide a reference for efficient QTL mapping of soybean.

### BRIEF DESCRIPTION OF THE DRAWING

FIG. 1 is a phenotypic distribution diagram showing a pod-shattering rate of a RIL population, where FIG. 1-E1 shows the pod-shattering rates of 260 RILs and two parents thereof in Heihe of Heilongjiang Province in 2015, and FIG. 1-E2 shows the pod-shattering rates of 260 RILs and two parents thereof in Heihe of Heilongjiang Province in 2016;
FIG. 2 shows the distribution of a SLAF tag and a polymorphic SLAF tag on a chromosome; where FIG. 2-A is a diagram showing the distribution of the SLAF tag, and FIG. 2-B is a diagram showing the distribution of the polymorphic SLAF tag;
FIG. 3 is a high-density genetic map of the RIL population;
FIG. 4 is a genetic map and genomic collinearity analysis;
FIG. 5 shows the mapping of QTLs on chromosome 8;
FIG. 6 is an analysis of the expression level of each soybean anti-pod-shattering candidate gene of the present invention; and
FIG. 7 is a violin plot of the qPD08-1 in offspring of 247RILs.

### DETAILED DESCRIPTION

The present invention provides a soybean anti-pod-shattering major QTL*qPD08-1*, the *qPD08-1* is mapped on the chromosome 8 of soybean at a physical position between 35560353-38482164; and
the genetic distance of 35560353-38482164 is 65.316-65.971 cM.

In the present invention, the soybean anti-pod-shattering major QTL has a LOD (logarithm of the odds) score of 7.171; an ADD (additive effect) value of -5.995, and a PVE (phenotypic variation explained) of 15.138%. The soybean anti-pod-shattering major QTLs were detected in both 2015 and 2016, and thus can be considered as stable QTLs.

The present invention provides a method for mapping the soybean anti-pod-shattering major QTL*qPD08-1*, including the following steps:
(1) constructing recombinant inbred lines of the fifth and sixth generations by using pod-shattering soybean and anti-pod-shattering soybean as parents, simultaneously sowing the pod-shattering soybean, the anti-pod-shattering soybean and the recombinant inbred line of the fifth-generation, and simultaneously sowing the pod-shattering soybean, the anti-pod-shattering soybean and the recombinant inbred line of the sixth-generation, to obtain samples of the pod-shattering soybean, the anti-pod-shattering soybean and the soybean recombinant inbred lines in two years;
(2) respectively extracting DNAs from samples of the pod-shattering soybean, the anti-pod-shattering soybean and the soybean recombinant inbred lines in the two years, to obtain genomic DNAs of the pod-shattering soybean, the anti-pod-shattering soybean and the soybean recombinant inbred lines;
(3) respectively constructing SLAF libraries by utilizing the genomic DNAs of the pod-shattering soybean, the anti-pod-shattering soybean and the soybean recombinant inbred lines, and separately sequencing the constructed SLAF libraries to obtain original sequencing data;
(4) filtering the original sequencing data to select 4-103 bp in the middle of each reads as the analysis data;
(5) aligning the analysis data to a reference genome, where each reads that has paired ends aligned to a reads at the same position is of the same SLAF tag, and then conducting polymorphic analysis of the SLAF tags according to the differences in the number of alleles and the gene sequences to obtain polymorphic SLAF tags;
(6) filtering the polymorphic SLAF tags to obtain screened polymorphic SLAF tags;
   where the filtering is to delete the polymorphic SLAF tags having the following conditions:
   a. a polymorphic SLAF tag having a parent sequencing depth below 10X;
   b. a polymorphic SLAF tag containing more than 5 SNP loci; and
   c. a polymorphic SLAF tag that is insufficient to cover 70% of genotype individuals in all progenies;
(7) aligning the screened polymorphic SLAF tags with a reference genome for mapping, where the screened polymorphic SLAF tags are mapped to 20 chromosomes, and using each chromosome as a linkage group to calculate a genetic distance between adjacent polymorphic SLAF tags on each linkage group, so as to obtain a high-density genetic map; and
(8) conducting QTL mapping of the pod-shattering phenotypes and pod-shattering traits of soybeans in the two years by employing an inclusive composite interval mapping method according to the high-density genetic map, so as to obtain the interval of the soybean anti-pod-shattering major QTLs.

In the present invention, recombinant inbred lines (RILs) of the fifth and sixth generations are constructed by using pod-shattering soybean and anti-pod-shattering soybean as parents; the pod-shattering soybean, the anti-pod-shattering soybean and the recombinant inbred line of the fifth-generation are sowed simultaneously; and the pod-shattering soybean, the anti-pod-shattering soybean and the recombinant inbred line of the sixth-generation are sowed simultaneously, to obtain samples of the pod-shattering soybean, the anti-pod-shattering soybean and the soybean recombinant inbred lines in two years.

In the present invention, the pod-shattering soybean is preferably used as the male parent; and the anti-pod-shattering soybean is preferably used as the female parent. The variety of the anti-pod-shattering soybean is preferably Heihe 43; and the variety of the pod-shattering soybean is preferably Heihe 18. In the present invention, the pod-shattering soybean and the anti-pod-shattering soybean are subjected to phenotype identification. The main evaluation index for the identification of soybean pod-shattering phenotypes is the pod-shattering rate. The pod-shattering rate = (the number of shattered pods/the number of total pods) × 100% (Preliminary analysis of the pod-shattering of soybean in Huanghuai area, Peng Yuhua, Soybean Science, 1991.). Asian Vegetable Research and Development Center (AVRDC) graded the pod-shattering phenotypes of soybean based on the pod-shattering rates, where the pod-shattering rate of 0% was set as grade 1 (high anti-pod-shattering), the pod-shattering rate between 0-10% was set as grade 2 (anti-pod-shattering), the pod-shattering rate between 11-25% was set as grade 3 (moderate anti-pod-shattering), the pod-shattering rate between 26-50% was set as grade 4 (moderate pod-shattering), and the pod-shattering rate above 50% was set as grade 5 (serious pod-shattering).

The present invention preferably employs an indoor drying method to determine the pod-shattering rate. In order to ensure the reliability of the data, it is avoided to touch the pods when samples are picked, so as to keep the consistency of the test materials before detection as far as possible. The indoor drying method includes the following steps: setting the temperature to 80°C and setting the drying time to 5 h as the optimum detection conditions for identifying the pod-shattering rate. The difference in the pod-shattering rate between Heihe 18 and Heihe 43 is extremely significant during manual detection. According to the AVRDC evaluation criteria, the Heihe 43 has the pod-shattering of Grade 2 (medium anti-pod-shattering), and the Heihe 18 has the pod-shattering of Grade 4 (medium pod-shattering), which is substantially consistent with the natural pod-shattering conditions.

The present invention has no specific limitation on the method for constructing the recombinant inbred lines of the fifth and sixth generations, and a method for constructing a recombinant inbred line which is well known in the art can be employed.

In the present invention, the samples of the pod-shattering soybean, the anti-pod-shattering soybean and the soybean recombinant inbred line of the fifth generation were planted in a geographical environment of Heilongjiang in 2015, and the samples of the pod-shattering soybean, the anti-pod-shattering soybean and the soybean recombinant inbred line of the fifth generation were planted in the geographical environment of Heilongjiang in 2016, so as to obtain samples of the pod-shattering soybean, the anti-pod-shattering soybean and the soybean recombinant inbred lines during the two years, respectively. The geographical environments of Heilongjiang in the two years are the same.

In the present invention, after the samples of the pod-shattering soybean, the anti-pod-shattering soybean and the soybean recombinant inbred lines in the two years are obtained, genomic DNAs are respectively extracted from each sample of the pod-shattering soybean, the anti-pod-shattering soybean and the soybean recombinant inbred lines in the two years, to obtain genomic DNAs of the pod-shattering soybean, the anti-pod-shattering soybean and the soybean recombinant inbred lines.

In the present invention, the samples of the pod-shattering soybean, the anti-pod-shattering soybean and the soybean recombinant inbred lines are planted in the same environment for two years, so as to obtain the samples of the pod-shattering soybean, the anti-pod-shattering soybean and the soybean recombinant inbred lines during the two years, respectively. In the present invention, preferably statistics of the respective pod-shattering phenotypes of the samples of the pod-shattering soybean, the anti-pod-shattering soybean and the soybean recombinant inbred lines during the two years is carried out for later QTL mapping.

In the present invention, the raw material for DNA extraction is preferably a leaf of a soybean sample. The present invention has no specific limitation on the DNA extraction method, and an extraction method well known in the art can be employed. In the present invention, the number of the samples of soybean recombinant inbred lines is preferably 200-300, and more preferably 260. The extracted DNA is preferably subjected to quality detection. The quality detection includes determination of DNA integrity and concentration of extracted DNA. The DNA integrity assay can be performed by employing detection through gel electrophoresis. The determination of the concentration of extracted DNA is preferably carried out by using a nucleic acid protein quantitative detector.

In the present invention, after the extracted DNA is obtained, a SLAF library is constructed by utilizing the genomic DNA of each of the samples of the pod-shattering soybean, the anti-pod-shattering soybean and the soybean recombinant inbred lines, and the constructed SLAF libraries are sequenced separately to obtain original sequencing data.

The present invention has no specific limitation on the method for constructing the SLAF library, and a solution for constructing a SLAF library well known in the art can be employed. In order to ensure accuracy during the construction of the SLAF library, it is preferred to use the soybean Williams 82 v2.1 genome (https://phytozome.jgi.doe.gov) as a reference genome for enzyme digestion. The type of the enzyme for enzyme digestion is preferably a combination of RsaI enzyme digestion and HaeIII enzyme digestion. In the present invention, the SLAF libraries are preferably constructed by conducting enzyme digestion of the genomic DNA of each sample to construct a library separately. A sequence having an enzyme-digested fragment length of 364-414 bp is defined as a SLAF tag, and it is predicted that 132,516 SLAF tags can be obtained in each library.

In the present invention, after the SLAF library is constructed, preferably Illumina HiSeq is used for sequencing of paired ends (125 bp). In the present invention, the sequencing depth of the SLAF library of the pod-shattering soybean is preferably 39.74 X; the sequencing depth of the SLAF library of the anti-pod-shattering soybean is preferably 34.87 X; and the sequencing depth of the SLAF library of the soybean recombinant inbred line is preferably 12.35X.

In order to evaluate the accuracy of the library-construction experiment, the present invention preferably uses *Oryza sativa* L japonica as a control to be subjected to the same treatment and participate in library construction and sequencing. The enzyme digestion efficiency is evaluated by the alignment efficiency of the control data, so as to judge the accuracy and effectiveness of the original sequencing data.

After the original sequencing data is obtained, the present invention filters the original sequencing data to select 4-103 bp in the middle of each reads as the analysis data.

In the present invention, the filtration criteria is preferably as follows: filtering out a reads containing a linker sequence; and filtering out a reads having a N content exceeding 10% of the length of the reads.

In the present invention, after the analysis data is obtained, the analysis data is aligned to the reference genome, where each reads that has paired ends aligned to a reads at the same position is of the same SLAF tag, and then polymorphic analysis of the SLAF tags is conducted according to the differences in the number of alleles and the gene sequences to obtain polymorphic SLAF tags.

In the present invention, the software used for aligning the analysis data to the reference genome is preferably the BWA software. The polymorphism analysis method is preferably carried out by using the BWA software.

In the present invention, in order to facilitate subsequent genetic analysis, the present invention conducts genotype encoding of the polymorphic SLAF tags. The genotype encoding method is preferably dividing the polymorphic tags into eight separation models (ab × cd, ef × eg, hk × hk, lm × ll, nn × np, aa × bb, ab × cc, and cc × ab) according to the biallelic encoding rule commonly used in genetics. RIL indicates that it is suitable for the type of aa × bb.

In the present invention, After the polymorphic SLAF tags are obtained, the polymorphic SLAF tags are filtered to obtain screened polymorphic SLAF tags;
where the filtering of the polymorphic SLAF tags is to delete the polymorphic SLAF tags having the following conditions:
a. a polymorphic SLAF tag having a parent sequencing depth below 10X;
b. a polymorphic SLAF tag containing more than 5 SNP loci; and
c. a polymorphic SLAF tag that is insufficient to cover 70% of genotype individuals in all progenies.

In the present invention, the filtering is beneficial for ensuring the improvement in the quality of the constructed genetic map.

In the present invention, after the screened polymorphic SLAF tags are obtained, the screened polymorphic SLAF tags are aligned to the reference genome for mapping, where the screened polymorphic SLAF tags are mapped to 20 chromosomes, and by using each chromosome as a linkage group, a genetic distance between adjacent polymorphic SLAF tags on each linkage group is calculated so as to obtain a high-density genetic map.

In the present invention, before the calculation of the genetic distance, the method further preferably includes performing second filtering on the polymorphic SLAF tags; where the criterion for the second filtering is to calculate an MLOD value between pairwise polymorphic SLAF tags, and then filter out polymorphic SLAF tags each with a MLOD value below 5. The second filtering facilitates obtaining of Markers, thereby depicting a high-density genetic linkage map.

In the present invention, QTL mapping is conducted according to the pod-shattering phenotypes and pod-shattering traits of soybeans in the two years by employing an inclusive composite interval mapping method, so as to obtain the interval of the soybean anti-pod-shattering major QTLs.

In the present invention, the software for the inclusive composite interval mapping method is preferably a R/qtl software. The inclusive composite interval mapping method performs threshold setting by using a permutation test for 1000 times, where firstly a LOD threshold corresponding to the confidence of 0.99 is considered, and if there is no mapped interval, then a LOD threshold corresponding to the confidence of 0.95 is considered; and if there is no mapped interval, then a LOD threshold corresponding to the confidence of 0.90 is considered. If there was still no result, the result of the permutation test was not considered, and the threshold was manually lowered to 3.0; and if there was no interval corresponding to 3.0, the threshold was lowered to 2.5. Finally, the LOD threshold is selected as 2.5 to determine if the QTL on the chromosome is related to the pod-shattering trait. The QTL obtained by the present invention is obtained when the LOD threshold is 2.5.

The present invention provides the application of the soybean anti-pod-shattering major QTL*qPD08-1* or the soybean anti-pod-shattering major QTL*qPD08-1* obtained by the mapping method in studying the genetic mechanism of the pod-shattering trait or screening molecular markers related to soybean yield. In the present invention, the molecular marker is a molecular marker of the CAPS type. Directing at the difference loci of the two parents in the QTL interval, an Indel molecular marker is developed for Indel loci, and the developed molecular marker is used to detect the RILs genotypes. The amplification primer for the Indel molecule marker includes a forward primer and a reverse primer; the forward primer includes the following nucleotide sequences: (1) a nucleotide sequence included as shown in SEQ ID NO. 1 of the Sequence Listing; and (2) a nucleotide sequence obtained by nucleotide substitution, deletion and addition on the basis of the condition (1); and the reverse primer includes the following nucleotide sequences: A. a nucleotide sequence included as shown in SEQ ID NO. 2 of the Sequence Listing; and B. a nucleotide sequence obtained by nucleotide substitution, deletion and addition on the basis of the condition A. 20 µL of the reaction system of the amplification primer for the Indel molecular marker includes 50 ng of genomic DNAs, a 10 × PCR buffer, 2 mmol/L of dNTPs, 2 mmol/L of a primer, and 1 U Taq polymerase. The amplification procedure of the amplification primer for the Indel molecular marker is: pre-denaturation at 94°C for 4 min, denaturation at 94°C for 30 s, annealing at 58°C for 40 s, extension at 72°C for 1 min, 34 cycles, and finally extension at 72°C for 10 min, and storage at 4°C.

The present invention provides the application of the soybean anti-pod-shattering major QTL*qPD08-1* in map-based cloning.

The present invention provides the application of the soybean anti-pod-shattering major QTL*qPD08-1* in transgenic breeding of a pod-shattering gene.

The present invention provides a soybean anti-pod-shattering gene obtained by screening based on the soybean anti-pod-shattering major QTL*qPD08-1*, including a MYC2 transcription factor coding sequence and a saponin synthetase coding sequence;
where the MYC2 transcription factor coding sequence includes the following nucleotide sequences:
(1) a nucleotide sequence as shown in SEQ ID No. 3 of Sequence Listing; and
(2) a nucleotide sequence obtained by nucleotide substitution, deletion and addition on the basis of the condition (1);
the saponin synthetase coding sequence includes the following nucleotide sequences:
(I) a nucleotide sequence as shown in SEQ ID No. 4 of Sequence Listing; and
(II) a nucleotide sequence obtained by nucleotide substitution, deletion and addition on the basis of the condition (I).

In the present invention, the screening method preferably includes the following steps:
the parent Heihe 43 is an anti-pod-shattering material, and the parent Heihe 18 is a pod-shattering material, and the soybean anti-pod-shattering gene is obtained by screening out a gene with significant difference in expression level between the two parents via analysis of the expression of genes within the QTL interval in the pods of the two parents during the R6 period.

In the present invention, the amplification primer of the MYC2 transcription factor coding sequence includes a forward primer and a reverse primer; in the Embodiments of the present invention, the nucleotide sequence of the forward primer is shown in SEQ ID No. 11 of Sequence Listing; and the nucleotide sequence of the reverse primer is shown in SEQ ID No. 12 of Sequence Listing. The saponin synthetase coding sequence includes a forward primer and a reverse primer; the nucleotide sequence of the forward primer is shown in SEQ ID No. 13 of Sequence Listing; and the nucleotide sequence of the reverse primer is shown in SEQ ID No. 14 of Sequence Listing. Amplification of the soybean anti-pod-shattering gene preferably adopts a two-step PCR reaction procedure: the first step being pre-denaturation: 95°C, 30 s; and the second step being PCR reaction: 95°C, 5 s; 60°C, 31 s, for 40 cycles.

The present invention also provides the application of the soybean anti-pod-shattering gene in selection of anti-pod-shattering varieties of soybean.

The soybean anti-pod-shattering major QTL*qPD08-1* and the mapping method and application thereof as provided by the present invention will be described in detail in connection with the following embodiments, but they should not be construed as limiting the claimed scope of the present invention.

### Embodiment 1

### Plant materials and identification of pod-shattering phenotypes

A mapping population containing 260 strains of RIL7 was configured by crossing between Heihe 43 as the female parent and Heihe 18 as the male parent. The obtained 260 strains of RIL7 of the fifth and sixth generations as well as the two parents thereof were simultaneously planted in a soybean test field at Heihe Branch of Heilongjiang Academy of Agricultural Sciences respectively in 2015 and 2016, where the planting environments of the two years were named E1 and E2, respectively. The parents were replicated for 3 times in 4 rows, and the materials of the offspring group were arranged in a random plot design, with a row length of 4 m, a row spacing of 66.7 cm, and a plant spacing of 5 cm. The plants were subjected to manual spot seeding. The female parent, Heihe 43, was the variety having the largest planting area in the early-maturing region of Heilongjiang Province. This highly anti-pod-shattering variety was prepared by hybrid breeding through a pedigree method, with the Heihe 18 as the female parent and the Heihe 23 as the male parent. The male parent, Heihe 18, was a variety of a type sensitive to pod shattering. Although the difference in agronomic traits between Heihe 18 and Heihe 43 was not significant, the pod shattering phenomenon of Heihe 18 was extremely obvious (Dezhi Han *et al.* 2015).

The pod-shattering rate was the main evaluation index for the identification of soybean pod-shattering phenotypes, where the pod-shattering rate = (the number of shattered pods/the number of total pods) × 100% (Peng Yuhua *et al.* 1991). Asian Vegetable Research and Development Center (AVRDC) graded the pod-shattering phenotypes of soybean based on the pod-shattering rates, where the pod-shattering rate of 0% was set as grade 1 (high anti-pod-shattering), the pod-shattering rate between 0-10% was set as grade 2 (anti-pod-shattering), the pod-shattering rate between 11-25% was set as grade 3 (moderate anti-pod-shattering), the pod-shattering rate between 26-50% was set as grade 4 (moderate pod-shattering), and the pod-shattering rate above 50% was set as grade 5 (serious pod-shattering).

This embodiment preferably employed an indoor drying method to determine the pod-shattering rate. The soybean pods at the full-ripe stage (R8) were cut with a scissor, and sealed in a ziplock bag (to prevent evaporation of water) and refrigerated in a refrigerator at 4°C to serve as a sample. In order to ensure the reliability of the data, it was avoided to touch the pods when samples were picked, so as to keep the consistency of the test materials before detection as far as possible. We set the oven temperature to 80°C and the drying time to 5 h as the optimum detection conditions for identifying the pod-shattering rate. In order to ensure the safety of the test, the experimental materials were placed in a high-temperature resistant wide-mouthed glass cup for the drying experiment. Before the start of the test, the oven was preheated to a treatment temperature in advance, the material was quickly taken out at the investigation time, and meanwhile the oven door was quickly closed (Dezhi Han *et al.* 2015). The difference in the pod-shattering rate between Heihe 18 and Heihe 43 was extremely significant during manual detection. According to the AVRDC evaluation criteria, the Heihe 43 had the pod-shattering of Grade 2 (medium anti-pod-shattering), and the Heihe 18 had the pod-shattering of Grade 4 (medium pod-shattering), which was substantially consistent with the natural pod-shattering conditions (Dezhi Han *et al.* 2015).

### Results

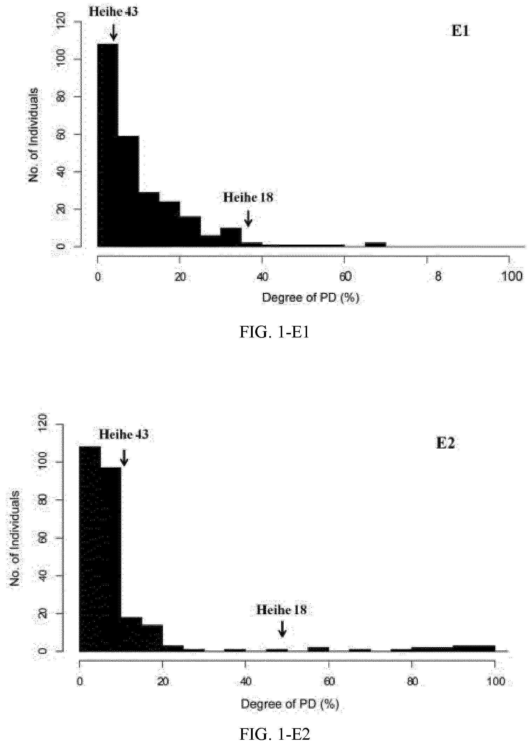

The pod-shattering rates of 260 RILs and two parents thereof in Heihe of Heilongjiang Province in 2015 (E1) and 2016 (E2) were identified (FIG. 1). During the two years, Heihe 18 showed a higher pod-shattering rate than that of Heihe 43, respectively of 37% versus 3%, and 47.03% versus 11.36%. During the two years, the distribution of pod-shattering rates of RILs was presented as a continuous distribution, but it did not conform to the normal distribution, and showed a skewed distribution biased to the female parent Heihe 43 (FIG. 1), indicating that the allele carried by the female parent Heihe 43 played the role of anti-pod-shattering. There was no obvious transgressive inheritance in the offspring. FIG. 1 showed the pod-shattering rates of the female parent Heihe 43 and the male parent Heihe 18 during the two years with arrows. E1 and E2 represented the planting environments at Heihe in 2015 and 2016, respectively.

### Embodiment 2

### DNA Extraction

Placed into a 2.0 mL centrifuge tube was an appropriate amount of soybean tender leaves (about 0.1 g), added with steel beads, and cooled in liquid nitrogen, thereafter the leaves were ground by a sample making machine and then stored at -80°C until use. The Soybean leaf DNA was extracted using a modified CTAB method (Saghaimaroof *et al.* 1984). The extracted DNA was subjected to mass detection by using 0.8% agarose gel electrophoresis and an ultraviolet spectrophotometer.

### Embodiment 3

### Constructing and sequencing of SLAF (Specific-locus amplified fragments) libraries

SLAF-library construction and sequencing were performed on 260 RILs and two parents thereof. Prediction of enzyme digestion was conducted by selecting the soybean Williams 82 v2.1 genome (https://phytozome.jgi.doe.gov) as a reference genome, where the combination of RsaI enzyme digestion and HaeIII enzyme digestion was selected to cleave the genomic DNA of each sample. A sequence having an enzyme-digested fragment length of 364-414 bp was defined as a SLAF tag, and it was predicted that 132,516 SLAF tags can be obtained. The obtained enzyme-digested fragments (SLAF tags) were subjected to the treatment of A addition at the 3' terminus, connection with Dual-index sequencing linker, PCR amplification, purification, sample mixing, and target-fragment selecting through gel excision, and then a sequencing library was constructed. The library was qualified and then sequenced for paired ends (125 bp) by using Illumina HiSeq. In order to evaluate the accuracy of the library-construction experiment, Oryza sativa L japonica was used as a control to be subjected to the same treatment and participate in library construction and sequencing. The enzyme digestion efficiency was evaluated by the alignment efficiency of the control data, so as to judge the accuracy and effectiveness. The SLAF tags were developed in parents and progenies by clustering of the reads, and the polymorphic SLAF tags were screened.

### Embodiment 4

### SLAF-seq polymorphic analysis and genotyping

The SLAF-seq data grouping and genotyping of the samples were performed according to the following procedure. The original sequencing read length of the SLAF-seq library was 125 bp at the end. In order to ensure the quality of information analysis, the original sequencing data was filtered. The criteria for filtering of the original data was as follows: filtering out a reads containing a linker sequence; filtering out a reads having a N content exceeding 10% of the length of the reads; where since the first few bps of the sequencing reads were the residue left by the enzyme digestion of fragments and had a lower sequencing quality at the end, 4-103 bp in the middle of the reads were selected for data analysis. The filtered sequencing reads were aligned to the reference genome by using the BWA software, where a reads that had paired ends aligned to a reads at the same position was of the same SLAF tag. Polymorphic analysis was conducted according to the differences in the number of alleles and the gene sequences, and the SLAF tags were classified. The SLAF tags were mapped to the reference genome, and the SLAF tags and polymorphic SLAF tags on different chromosomes were counted. A distribution map of the SLAF tags and the polymorphic SLAF tags on the chromosomes was drawn according to the distribution of the SLAF tags on the chromosomes.

In order to facilitate subsequent genetic analysis, in this embodiment genotype encoding of the polymorphic tags was conducted, and the polymorphic tags were divided into eight separation models (ab × cd, ef × eg, hk × hk, lm × ll, nn × np, aa × bb, ab × cc, and cc × ab) according to the biallelic encoding rule commonly used in genetics. The type of aa × bb was suitable for inbred populations (such as F2, RIL, DH), and the remaining markers were suitable for hybrid populations. The population used in the present invention was RIL, the parental genotypes were aa (male parent) and bb (female parent), and the progeny genotype is ab, indicating that the sample was heterozygous in terms of the encoding type of the marker, where one of genotypes was derived from the male parent, and the other one of the genotypes was derived from the female parent. In order to ensure the quality of the genetic map, the polymorphic SLAF tags were filtered according to the following rules: 1) filtering out a polymorphic SLAF tag having a parental sequencing depth below 10X; and 2) filtering out a polymorphic SLAF tag for which the number of SNPs was greater than 5. Since the sequencing length of the SNP tag was 200 bp, occurring of too many SNPs was considered as a high-frequency variation region in sequencing. 3) integrity filtering. Markers that at least cover more than 70% of genotype individuals in all progenies were screened. The loci of which the parental information was missing was filtered out based on the detection results of the parental genotypes, and finally the obtained polymorphic SLAF tags were used to construct a high-density genetic map. The content of this part was entrusted to Biomarker Technologies to complete.

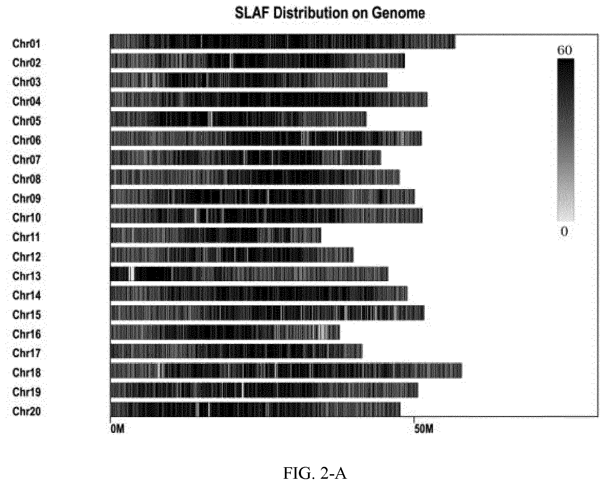

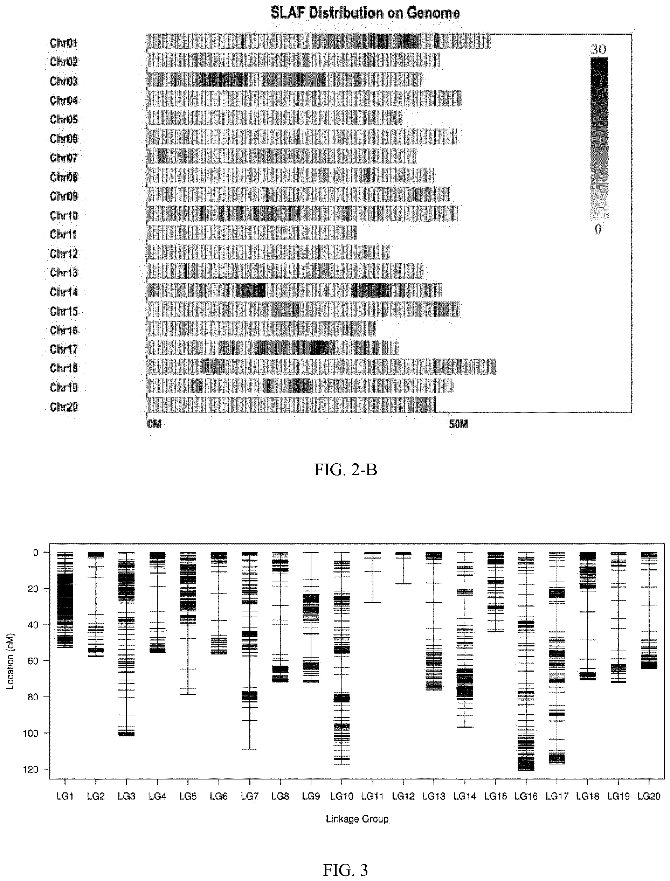

Genotyping was conducted on the two parents and the RIL population using SLAF-Seq. The enzyme digestion efficiency was 89.82%, indicating that the enzyme digestion efficiency was normal. The number of control sequencing reads obtained by us for evaluating the accuracy of experimental library construction was 398,386. The control sequencing reads was aligned to the reference genome with an alignment efficiency of 86.29%, and the alignment efficiency was substantially normal. The average Q30 of sequencing was 80.40%, and the average GC content was 38.12%. We had obtained 364,461 SLAF tags in total. The SLAF-tag sequencing depths of the two parents, Heihe 43 and Heihe 18, were 39.74 X and 34.87 X, respectively, and the numbers of SLAF tags were 195,279 and 205,644, respectively. The average SLAF-tag sequencing depth of the progeny was 12.35X. Polymorphic analysis was conducted on the obtained SLAF tags according to the differences in the number of alleles and the gene sequences, and 3 types of SLAF tags were obtained in total: polymorphic, non-polymorphic, and repetitive SLAF tags. The types of the tags developed by the SLAF technology were mainly SNP tags and Indel tags. There were 24,249 polymorphic SLAF tags in total, and the polymorphism ratio reached 6.65%. Genotyping was conducted on the obtained 24,249 polymorphic SLAF tags, and 11,028 tags were successfully genotyped. The material used in this study was the RIL₇ population. Loci were selected such that the parental genotypes were homozygous and there was polymorphism between parents. The polymorphic tags of aa × bb type were selected as effective tags conforming to the characteristics of this population. The effective polymorphism of the genetic map constructed in this study was 2.68%. After filtering and quality evaluation, 5,227 SLAF tags available for construction of the high-density genetic map were obtained, and the types of the SLAF tags were all the aa × bb type. A distribution map of all of the SLAF tags and the polymorphic SLAF tags on the chromosomes was drawn according to the distribution of the SLAF tags on the chromosomes (FIG. 2). In FIG. 2, the horizontal axis was the length of the chromosome, where each yellow band represented a chromosome, the genome was divided based on the size of 1 M, and the color was darker when the number of SLAF tags in each window was more, and the color was lighter when the number of SLAF tags in each window was fewer; the darker area in the figure was the area where the SLAF tags were concentrated. FIG. 2-A was a diagram showing the distribution of the SLAF tag, and FIG. 2-B was a diagram showing the distribution of the polymorphic SLAF tag.

### Embodiment 5

### Construction of high-density genetic map

After the obtained polymorphic SLAF tags were aligned to the reference genome for mapping, the SLAF tags were mapped to 20 chromosomes, and the tags each having a MLOD (the modified logarithm of odds) value relative to other SLAF tags below 5 were filtered out by calculating the MLOD value between pairwise tags. The screened tags were the Markers. Each chromosome was a linkage group. By using the linkage group as a unit, a linear arrangement of Markers in the linkage group was obtained by analyzing with a HighMap composition software, and the genetic distance between adjacent Markers was estimated, so as to finally obtain the high-density genetic map.

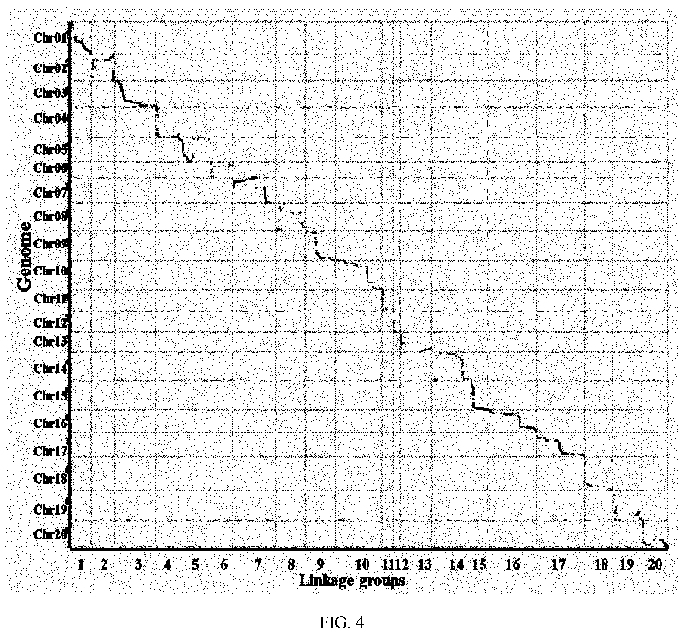

The screened 5,227 SLAF tags were divided into 20 linkage groups by mapping to the reference genome. by filtering out the tags each having a MLOD value relative to other SLAF tags below 5, a total of 4,593 Markers were obtained, and finally a genetic map with the total map distance of 1,478.86 cM was obtained (Fig. 3). The average genetic distance of the tags on the chromosomes was 0.53 cM. The average length of each chromosome was 73.94 cM. The chromosome with the highest number of tags was Gm03, which had 706 tags and a total length of 101.35 cM. The chromosome with the lowest number of tags was Gm12, which had 17 tags and a total length of 17.27 cM. Furthermore, the proportion of the inter-marker gap of less than 5 cM was found to be 95.72% (Table 1). The collinearity analysis of the positions of the Markers on the genome and the genetic map showed that the orders of most of the tags on the 20 chromosomes were consistent with those of the genome, indicating that the collinearity was good and the calculation accuracy of the genetic recombination rate was high (FIG. 4). Therefore, conducting gene annotation in the QTL interval was reliable. In FIG. 4, the horizontal axis was the genetic distance of each linkage group, and the vertical axis was the physical length of each linkage group, where the collinearity relationship of the Markers in the genome and the genetic map was represented in the form of scatter. The more diagonal relationship presented by the markers indicated that the collinearity between the genetic map and the genome was better. Different colors indicated different chromosomes or linkage groups. The content of this part was entrusted to Biomarker Technologies to complete.

The segregation distortion phenomenon was ubiquitous. The Markers of the present invention contained 4,394 segregation distortion markers, accounting for 95.67% of the total number of markers, where the number of tags biased to the male parent Heihe 18 was 1,611, and the number of tags biased to the female parent Heihe 43 was 2,783. Polymorphic markers with partial segregation distortion (chi-square test, *P* < *0.01*) were selected for map construction.

**Table 1 Information of high-density genetic map**

| Chromosome | Number of tags^{b} | Total map distance (cM) | Average map distance (cM) Average map distance | Gaps ≤ 5 | Maximum Gap (cM) |
|---|---|---|---|---|---|
| 1 | 489 | 52.51 | 0.11 | 100% | 2 |
| 2 | 66 | 57.71 | 0.89 | 95.38% | 20.61 |
| 3 | 706 | 101.35 | 0.14 | 99.57% | 9.75 |
| 4 | 103 | 55.26 | 0.54 | 96.08% | 13.8 |
| 5 | 135 | 78.59 | 0.59 | 97.76% | 16.95 |
| 6 | 64 | 56.2 | 0.89 | 95.24% | 15.12 |
| 7 | 393 | 108.94 | 0.28 | 97.19% | 17.33 |
| 8 | 115 | 71.71 | 0.63 | 97.37% | 16.47 |
| 9 | 96 | 71.87 | 0.76 | 97.89% | 14.79 |
| 10 | 463 | 117.27 | 0.25 | 99.35% | 10.38 |
| 11 | 44 | 27.83 | 0.65 | 93.02% | 17.33 |
| 12 | 17 | 17.27 | 1.08 | 87.50% | 13.75 |
| 13 | 111 | 76.76 | 0.7 | 82.73% | 14.16 |
| 14 | 450 | 96.77 | 0.22 | 99.11% | 12.97 |
| 15 | 338 | 43.79 | 0.13 | 100% | 4.63 |
| 16 | 272 | 120.6 | 0.45 | 97.79% | 6.81 |
| 17 | 399 | 117.1 | 0.29 | 98.74% | 15.84 |
| 18 | 157 | 70.73 | 0.45 | 97.44% | 15.34 |
| 19 | 84 | 72.31 | 0.87 | 85.54% | 12.45 |
| 20 | 91 | 64.29 | 0.71 | 96.67% | 14.41 |
| Total | 4,593 | 1,478.86 | 0.53 | 95.72% | 20.61 |

### Embodiment 6

### QTL mapping of the pod-shattering trait of soybean

QTL mapping of pod-shattering related traits was carried out according to the soybean pod-shattering phenotypes in two environments by using the inclusive composite interval mapping method of a R/qtl software package. Threshold setting was performed by using a permutation test for 1000 times, where firstly a LOD threshold corresponding to the confidence of 0.99 was considered, and if there was no mapped interval, then a LOD threshold corresponding to the confidence of 0.95 was considered; and if there was no mapped interval, then a LOD threshold corresponding to the confidence of 0.90 was considered. If there was still no result, the result of the permutation test was not considered, and the threshold was manually lowered to 3.0; and if there was no interval corresponding to 3.0, the threshold was lowered to 2.5. Finally, the LOD value was selected as 2.5 to determine if the QTL on the chromosome was related to the pod-shattering trait. The content of this part was entrusted to Biomarker Technologies to complete.

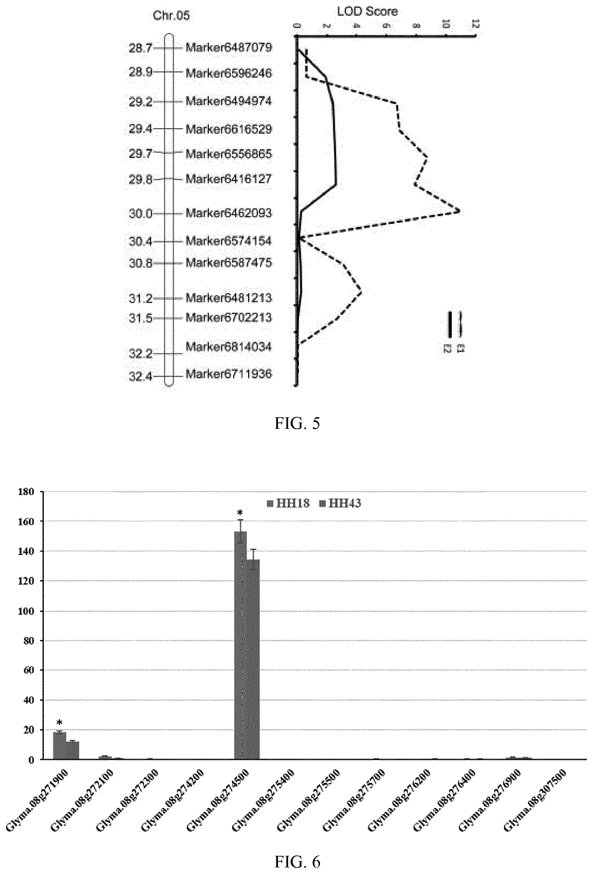

The QTLs related to the anti-pod-shattering of RILs in two environments were detected by using the inclusive composite interval mapping method of a R/qtl software package. In this study, we detected a total of 4 related QTLs respectively located on the chromosomes 1, 5, and 8 (FIG. 5), and the explained phenotypic variation rate was ranged from 8.37% to 24.443% (Table 2). Among the 4 QTLs, each of 3 QTLs (qPD01, qPD05, qPD08-1) explained a higher phenotypic variation rate (> 10%), where the QTLs (qPD05, qPD08-1) located on chromosomes 5 and 8 could be detected both in the two years, and thus can be considered as stable QTLs in the population of this study. The QTL (qPD01) located on chromosome 1 was only detected in 2016, which explained the phenotypic variation rate of 24.443% (Table 2). The additive effects of these QTLs were all negative values, indicating that the superior alleles were all derived from the female parent Heihe 43, and the alleles carried by the female parent Heihe 43 played a role in anti-pod-shattering.

**Table 2 Soybean pod-shattering QTLs in different environments**

| QTL^{a} | Env^{b} | Chr^{c} | Flanking marker^{d} | Interval^{e}(cM) | LOD^{f} | ADD^{g} | PVE^{h}(%) |
|---|---|---|---|---|---|---|---|
| *qPD08-1* | E1, E2 | 8 | 35560353 to 38482164 | 65.316 to 65.971 | 3 | -12.723 | 19.611 |

| | | | | | | | |
|---|---|---|---|---|---|---|---|
| a the QTL name was a combination of soybean pod-shattering; b Environment: E1 indicated the planting environment at Heihe in 2015, and E2 indicated the planting environment at Heihe in 2016; c Chromosome; d markers on both sides, markers on the left and right sides of the QTL; e interval, a confidence interval between two markers; f LOD, logarithm of odds; g additive effect; h the phenotypic variation rate explained by each QTL. | | | | | | | |

### Embodiment 7

### Development and application of molecular markers in the interval of QTLs

Directing at the difference loci of the two parents in the QTL interval, in this embodiment an Indel molecular marker was developed for Indel loci, and the developed molecular marker was used to detect the RILs genotypes. An Indel marker was developed according to the Indel loci in the *qPD08-1.* PCR primers were designed on both sides of a candidate Indel marker using a Primer 3.0 software (Table 3).

Using the genomic DNAs of the two parents and the 260 parts of RILs as templates, 20 µL of a PCR reaction system includes 50 ng of genomic DNAs, a 10x PCR Buffer, 2 mmol/L of dNTPs, 2 mmol L⁻¹ of a primer and 1 U Taq polymerase (TransGen Biotech). The PCR amplification procedure was: pre-denaturation at 94°C for 4 min, denaturation at 94°C for 30 s, annealing at 58°C for 40 s, extension at 72°C for 1 min, 34 cycles, and finally extension at 72°C for 10 min, and storage at 4°C. The PCR product was detected by 1.5% agarose gel electrophoresis. According to the (NEW ENGLAND BioLabs) enzyme digestion method, 10 µL of an enzyme digestion system including 5 µL of the PCR product, 0.2 µ 10 U/µL of an enzyme, 1.5 µL of a buffer (NEB, www.neb.com/), and 3.3 µL of ddH₂O, was used to conduct enzyme digestion in a constant-temperature water bath at 37°C for 40 minutes. The digested product was detected by 2% agarose gel electrophoresis. The CAPS/dCAPS-labeled digested product was detected by 2% agarose gel electrophoresis; and the denatured Indel-labeled PCR product was separated by using 7% denatured polyacrylamide gel electrophoresis (PAGE).

**Table 3 Information of primer sequence**

| QTL | Typ e of mar ker | Mutat ion site | Physic al locatio n | Endonuc lease | Sequence of Primer | Fragm ent Lengt h (bp) | Length of enzyme-di gested fragment |
|---|---|---|---|---|---|---|---|
| | | | | | (5'-3') | | (bp) |
| *qPD0 8-1* | Inde l | GTT T/G | 36811 354 | - | | 422 | - |
| | | | | | | | |

In this embodiment, directed to the difference loci in the QTL interval, PCR amplification of the two parents was performed by using the designed primers (Table 3), and individual PCR products each having a size similar to the length of the target fragment were obtained. An analysis of alignment to the Williams 82 reference sequence revealed that, the two parents had differences in the target Indel position, which was consistent with the prediction. After the PCR products produced from the Indel marker in the *qPD08-1* interval were separated by the denatured polyacrylamide gel electrophoresis, two types of banding patterns were detected, one had the DNA fragment size of 422 bp and the allelic variation of GTTT; and the other had the DNA fragment size of 419 bp and the allelic variation of G. It had been determined that the identification of the two parents on the 4 SNP and Indel loci was correct, indicating that the developed Indel marker could be used for the genotype identification of the 260 parts of RILs progenies.

### Embodiment 8

### Application of molecular markers in the interval of the QTLs on chromosome 8

The developed molecular marker was used to identify the genotypes of 36 parts of cultivated soybeans and 36 parts of wild soybeans. The primer sequences were primers used in the development of the molecular marker. Using the genomic DNAs of the 36 parts of cultivated soybeans and the 36 parts of wild soybeans as templates, 20 µL of a PCR reaction system included 50 ng of genomic DNAs, a 10x PCR Buffer, 2 mmol/L of dNTPs, 2 mmol/L of a primer and 1 U Taq polymerase (TransGen Biotech). The PCR amplification procedure was: pre-denaturation at 94°C for 4 min, denaturation at 94°C for 30 s, annealing at 58°C for 40 s, extension at 72°C for 1 min, 34 cycles, and finally extension at 72°C for 10 min, and storage at 4°C.

After the PCR products produced from the Indel marker in the qPD08-1 interval were separated by the denatured polyacrylamide gel electrophoresis, two types of banding patterns were detected, one had the DNA fragment size of 422 bp and the allelic variation of GTTT; and the other had the DNA fragment size of 419 bp and the allelic variation of G. The female parent Heihe 43 had the DNA fragment size of 419 bp and the allelic variation of G; and the male parent Heihe 18 had the DNA fragment size of 422 bp and the allelic variation of GTTT.

As identified, among the 36 parts of cultivated soybeans, 7 parts of the materials had the genotype of GTTT, and 29 parts of the materials had the genotype of G. Among the 36 parts of wild soybeans, 1 part of the materials had the genotype of GTTT, and 35 parts of the materials had the genotype of G.

The genotypes of 18 parts of fresh edible soybeans were identified using the developed molecular marker. As identified, among the 18 parts of fresh edible soybeans, 2 parts of the materials had the genotype of GTTT, and 16 parts of the materials had the genotype of G.

### Embodiment 9

The parent Heihe 43 was an anti-pod-shattering material, and the parent Heihe 18 was a pod-shattering material, and the candidate genes were further screened at a transcriptome level by screening out a gene with significant difference in expression level between the two parents via analysis of the expression of genes within the QTL interval in the pods of the two parents during the R6 period.

The specific implementation was as follows:
the CDS sequences of the genes in the mapping interval were extracted at Phytozome (https://phytozome jgi.doe.gov/pz/portal.html#!search?show=KEYWORD&method=Org_Gmax), and the expression analysis primer was designed by using a PrimerS software (Table 1). The RNA of a soybean pod tissue was extracted according to the operation instruction of RNAprep Pure Plant Total RNA Extraction Kit (Cat No.: DP432) of TIANGEN Biotech (Beijing) Co.,Ltd. The RNA of the pod tissue was reverse-transcribed into a double-stranded cDNA by utilizing the operation instruction of a FastQuant RT Kit (Cat No.: KR106) reverse transcription kit, and the expression analysis was completed on a fluorescence quantitative PCR instrument ABI7300. The specific reaction system refers to a SYBR Premix Ex Taq (Cat No.: RR420A) of Takara, and a two-step PCR reaction procedure was adopted: the first step being pre-denaturation: 95°C, 30 s; and the second step being PCR reaction: 95°C, 5 s; 60°C, 31 s, for 40 cycles.

**Table 4 List of information about qRT-PCR primers**

| Gene ID | Primer sequence (5' to 3') | Sequence No. | Amplification Length (bp) |
|---|---|---|---|
| *Glyma.08g271900* | F: catttttcagaaccccgatctg | SEQ ID No.5 | 229 |
| | R: ggtttcgaaatgcatcgtctta | SEQ ID No.6 | |
| *Glyma.08g272100* | F: ttctccaacctctcagtttctc | SEQ ID No.9 | 84 |
| | R: aacagtggtcgataacaagact | SEQ ID No.10 | |
| *Glyma.08g272300* | F: tatcagactcaatgggaaaacgg | SEQ ID No.11 | 103 |
| | R: ttgcagataaaaccgcgccaaaa | SEQ ID No.12 | |
| *Glyma.08g274200* | F: gtatcagcagcatacccttttg | SEQ ID No.13 | 203 |
| | R: ctgtctttctcctcaactttgc | SEQ ID No.14 | |
| *Glyma.08g274500* | F: gcagttgttgcaattggaagatgtt | SEQ ID No.7 | 93 |
| | R: gcaccactcacaggtagggatag | SEQ ID No.8 | |
| *Glyma.08g275400* | F: gaatccgcattcaacatgaaga | SEQ ID No.15 | 117 |
| | R: ggattgaatggagttgacatcg | SEQ ID No.16 | |
| *Glyma.08g275500* | F: aaaggtttttggccctgtattc | SEQ ID No.17 | 128 |
| | R: taggaaaatcgtaaggccaact | SEQ ID No.18 | |
| *Glyma.08g275700* | F: taggagacaaggtgggtgtggga | SEQ ID No.19 | 167 |
| | R: gctatacactgtgatcccagcacag | SEQ ID No.20 | |
| *Glyma.08g276200* | F: tcgtgatgtggttatttgaacaa | SEQ ID No.21 | 113 |
| | R: aaggcgaagagatggaagagagt | SEQ ID No.22 | |
| *Glyma.08g276400* | F: tgatgttataaatcccgcaacg | SEQ ID No.23 | 84 |
| | R: gcactaactgcagctttaaact | SEQ ID No.24 | |
| *Glyma.08g276900* | F: gtgctttaagccaagtctttca | SEQ ID No.25 | 90 |
| | R: gttatcggcttcggtttttctt | SEQ ID No.26 | |
| *Glyma.08g307500* | F: aatagtgctctctctcacacac | SEQ ID No.27 | 193 |
| | R: gtgctcaaacttgcagttgtaa | SEQ ID No.28 | |
| *GmActin* | F: ggtggttctatcttggcatc | SEQ ID No.29 | 246 |
| | R: ctttcgcttcaataacccta | SEQ ID No.30 | |

The expression analysis results were shown in FIG. 6. As can be seen from FIG. 6, in this interval the expression levels of two genes, i.e., Glyma.08g271900 (the MYC2 transcription factor coding sequence) and Glyma.08g274500 (the saponin synthetase coding sequence) in the pods of the anti-pod-shattering and pod-shattering parents were significantly different, so that the two genes were taken as candidate genes for controlling the pod-shattering in this interval, and could provide gene resources for subsequent controlling of the cloning of the anti-pod-shattering gene.

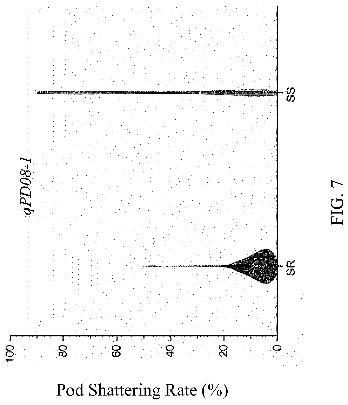

The variance analysis of phenotype and genotype was carried out on 247 RILs. The violin plot of the qPD08-1 in offspring of 247 RILs was shown in FIG. 7. The results showed that there were 211 individuals carrying the anti-pod-shattering genotype, with an average pod-shattering rate of 7.6%; and there were 36 individuals carrying the pod-shattering genotype, with an average pod-shattering rate of 29.05%. There was a very significant difference in pod-shattering rate between individuals of the two genotypes (*P* = 0.000082).

## Claims

1. Primers for amplifying the molecule marker of soybean anti-pod-shattering major QTLqPD08-1, wherein the molecule marker is an Indel molecule marker; the primers for amplifying the Indel molecule marker comprise a forward primer and a reverse primer; the forward primer has a nucleotide sequence as shown in SEQ ID NO. 1 of the Sequence Listing; and the reverse primer has a nucleotide sequence as shown in SEQ ID NO. 2 of the Sequence Listing.

2. Use of the primers according to claim 1 in the genetic mechanism of the pod-shattering trait or the screening for molecular markers related to soybean yield;
wherein the major QTL is mapped on the chromosome 8 of soybean at a physical position between 35560353-38482164; and the genetic distance of 35560353-38482164 is 65.316-65.971 cM; selecting the soybean Williams 82 v2.1 genome as a reference genome.

3. Use of the primers according to claim 1 in map-based cloning.

4. A soybean anti-pod-shattering gene obtained by screening based on the soybean anti-pod-shattering major QTLqPD08-1, wherein the soybean anti-pod-shattering gene is a MYC2 transcription factor coding sequence or a saponin synthetase coding sequence;
wherein the nucleotide sequence of the MYC2 transcription factor is set forth in SEQ ID No. 3 of Sequence Listing; and
the nucleotide sequence of the saponin synthetase is set forth in SEQ ID No. 4 of Sequence Listing.

## Patentansprüche

1. Primer zum Amplifizieren des Molekülmarkers von Major QTLqPD08-1 zum Schutz vor dem Hülsenplatzen bei Sojabohnen, wobei der Molekülmarker ein Indel-Molekülmarker ist; die Primer zum Amplifizieren des Indel-Molekülmarkers umfassen einen Vorwärtsprimer und einen Rückwärtsprimer; der Vorwärtsprimer weist eine Nukleotidsequenz auf, wie gezeigt in SEQ ID NO. 1 des Sequenzprotokolls; und der Rückwärtsprimer weist eine Nukleotidsequenz auf, wie gezeigt in SEQ ID NO. 2 des Sequenzprotokolls.

2. Verwendung der Primer nach Anspruch 1 im genetischen Mechanismus des Merkmals zum Schutz vor dem Hülsenplatzen oder beim Screening auf molekulare Marker, die mit der Sojabohnenausbeute zusammenhängen;
wobei der Major-QTL auf dem Chromosom 8 der Sojabohne an einer physischen Position zwischen 35560353-38482164 kartiert ist; und der genetische Abstand von 35560353-38482164 beträgt 65.316-65.971 cM; Auswählen des Sojabohnen-Williams 82 v2.1-Genoms als Referenzgenom.

3. Verwendung der Primer nach Anspruch 1 bei der kartenbasierten Klonierung.

4. Gen zum Schutz vor dem Hülsenplatzen bei Sojabohnen, erhalten durch Screening auf der Grundlage des Major QTLqPD08-1 zum Schutz vor dem Hülsenplatzen bei Sojabohnen, wobei das Gen zum Schutz vor dem Hülsenplatzen bei Sojabohnen eine MYC2-Transkriptionsfaktor-kodierende Sequenz oder eine Saponinsynthetase-kodierende Sequenz ist;
wobei die Nukleotidsequenz des MYC2-Transkriptionsfaktors in SEQ ID Nr. 3 des Sequenzprotokolls aufgeführt ist; und
die Nukleotidsequenz der Saponinsynthetase ist in SEQ ID Nr. 4 des Sequenzprotokolls aufgeführt.

## Revendications

1. Amorces pour l'amplification du marqueur moléculaire du QTLqPD08-1 majeur anti-égrenage de gousses de graines de soja, dans lequel le marqueur moléculaire est un marqueur moléculaire Indel; les amorces pour l'amplification du marqueur moléculaire Indel comprennent une amorce directe et une amorce inverse; l'amorce directe a une séquence nucléotidique telle qu'indiquée dans SEQ ID NO. 1 de la liste des séquences; et l'amorce inverse a une séquence nucléotidique telle qu'indiquée dans SEQ ID NO. 2 de la liste des séquences.

2. Utilisation des amorces selon la revendication 1 dans le mécanisme génétique du caractère d'égrenage des gousses ou dans le criblage de marqueurs moléculaires liés au rendement du soja;
dans lequel le QTL majeur est cartographié sur le chromosome 8 de la graine de soja à une position physique entre 35560353-38482164; et la distance génétique de 35560353-38482164 est de 65,316-65,971 cM; en sélectionnant le génome Williams 82 v2.1 de graine de soja comme génome de référence.

3. Utilisation des amorces selon la revendication 1 dans le clonage positionnel.

4. Gène anti-égrenage de gousses de graines de soja obtenu par criblage basé sur le QTLqPD08-1 majeur anti-égrenage de gousses de graines de soja, dans lequel le gène anti-égrenage de gousses de graines de soja est une séquence de codage du facteur de transcription MYC2 ou une séquence de codage de la saponine synthétase;
dans lequel la séquence de nucléotides du facteur de transcription MYC2 est présentée dans SEQ ID No. 3 de la liste des séquences; et
la séquence nucléotidique de la saponine synthétase est présentée dans SEQ ID No. 4 de la liste des séquences.
